# EUROPÄISCHE PATENTSCHRIFT

(11) **EP 0 328 913 B1**
(45) Veröffentlichungstag und Bekanntmachung des Hinweises auf die Patenterteilung: **08.06.1994**
(21) Anmeldenummer: 89101335.1
(22) Anmeldetag: 26.01.1989
(51) Int. Cl.: C07D 513/04, A61K 31/495

(54) **Neue basisch substituierte 5-Halogen-Thienoisothiazol-3(2H)-on-1,1-dioxide, ein Verfahren zu ihrer Herstellung und diese Verbindungen enthaltende pharmazeutische Präparate**
Basically substituted 5-halothienoisothiazol-3(2H)-one-1,1-dioxides, process for their preparation and pharmaceutical compositions containing them
5-Halothiénoisothiazole-3(2H)-one-1,1-dioxydes à substituant basique, procédé pour leur préparation et compositions pharmaceutiques les contenant

(30) Priorität: 18.02.1988 AT 390/88
(43) Veröffentlichungstag der Anmeldung: 23.08.1989
(73) Patentinhaber: Chemisch Pharmazeutische Forschungsgesellschaft m.b.H., 4021 Linz (AT)
(72) Erfinder: Binder, Dieter, Dr., A-1190 Wien (AT); Rovenszky, Franz, Dipl.-Ing.Dr., A-2460 Bruck a.d.Leitha (AT)

(56) Entgegenhaltungen:
- EP-A- 0 129 128
- EP-A- 0 213 295
- EP-A- 0 220 051

## Beschreibung

Die Erfindung betrifft neue basisch substituierte 5-Halogen-Thienoisothiazol-3(2H)-on-1,1-dioxide, ein Verfahren zu ihrer Herstellung, diese Verbindungen enthaltende pharmazeutische Präparate und deren Verwendung in Medikamenten zur Behandlung von Angstzuständen.

Aus der EP-A 220 051 sind anxiolytisch wirksame 4-(1-Piperazinyl)-butyl-thieno- oder benzo-isothiazol-3(2H)-one bekannnt, wobei die Thieno-Verbindungen am Thiophenring unsubstituiert sind. Es wurden nun neue, am Thiophenring in Position 5 durch Halogen substituierte Thieno-iso-thiazol-3(2H)-one gefunden, die die in der EP-A 220 051 genannten unsubstituierten Thiophenverbindungen mehrfach in ihrer Wirksamkeit übertreffen. Ähnliche Produkte sind in der EP-A-0 129 128 beschrieben.

Gegenstand der Erfindung sind daher neue Verbindungen der Formel I des Formelblattes, in der
R₁ Wasserstoff, (C₁ - C₄)-Alkyl oder Halogen,
R₂ Halogen und
n eine ganze Zahl 2 bis 6 darstellt, ihre pharmazeutisch verträglichen Säureadditionssalze, ein Verfahren zu ihrer Herstellung, pharmazeutische Präparate, die diese Verbindungen enthalten sowie deren Verwendung in Medikamenten zur Behandlung von Angstzuständen.

Der Ausdruck (C₁ - C₄)-Alkyl umfaßt alle geradkettigen und verzweigten gesättigten Kohlenwasserstoffreste mit 1 - 4 Kohlenstoffatomen wie beispielsweise Methyl, Ethyl, Propyl, Isopropyl, tert. Butyl. Unter Halogen ist Chlor, Brom und Jod zu verstehen.

Von den Verbindungen der Formel I sind jene bevorzugt, in denen
R₁ Wasserstoff,
R₂ Chlor und
n eine ganze Zahl 4 bedeutet.

Besonders bevorzugt ist die Verbindung
5-Chlor-2-(4-(4-(2-pyrimidinyl)-1-piperazinyl)butyl)thieno-(2,3-d)isothiazol-3(2H)-on-1,1-dioxid.

Die Verbindungen der Formel I und ihre Salze werden dadurch hergestellt, daß man eine Verbindung der Formel II des Formelblattes, in der
R₂ und n wie oben definiert sind und X Halogen bedeutet, mit einer Verbindung der Formel III des Formelblattes, in der R₁ Wasserstoff, (C₁ - C₄)-Alkyl oder Halogen bedeutet, umsetzt und die erhaltene Base der Formel I erwünschtenfalls in ein pharmazeutisch verträgliches Säureadditionssalz überführt.

Die Umsetzung einer Verbindung der Formel II des Formelblattes mit einer Verbindung der Formel III des Formelblattes findet in einem unter Reaktionsbedingungen inerten Verdünnungsmittel statt. Beispielsweise löst man eine Verbindung der Formel II in einem organischen Verdünnungsmittel wie beispielsweise DMF, DMSO, Chlorbenzol, Diethylcarbonat oder Aceton, tropft eine Lösung einer Verbindung der Formel III im gleichen Lösungsmittel zu und läßt unter Rühren fertig reagieren. Die Reaktionszeit liegt dabei zwischen 30 Minuten und 15 Stunden bei einer Temperatur von 20 bis 150 °C, bevorzugt bei 50 bis 70 °C, wobei höhere Reaktionstemperaturen kürzere Reaktionszeiten bedingen und umgekehrt.

Die Aufarbeitung der erhaltenen Verbindungen der Formel I erfolgt in der üblichen Weise durch Eindampfen, Ausfällen, Fällen als Salz, Extrahieren, Umkristallisieren oder durch Säulenchromatographie. Bewährt hat sich hierbei die Extraktion des Rohproduktes aus der wässrigen Phase mittels Methylenchlorid. Da die freien Basen der Formel I meist nur schwer reinigbare Substanzen sind, empfiehlt es sich, die Reinigung über gut kristallisierende Säureadditionsverbindungen vorzunehmen.

Dazu löst man die freie Base in einem geeigneten Lösungsmittel, z.B. in einem niederen Alkohol oder Ether oder in Aceton und gibt eine mindestens äquivalente Menge Protonensäure wie beispielsweise HCl, HBr, H₂SO₄, Weinsäure oder Zitronensäure zu. Man dampft nötigenfalls ein und kristallisiert aus Methanol, Ethanol, Isopropanol, n-Propanol oder Aceton, gegebenenfalls unter Zusatz von Ether, um.

Diese Säureadditionssalze können dann in an sich bekannter Weise, z.B. mit Alkalien oder Ionenaustauschern in die freien Basen übergeführt werden, von denen sich durch Umsetzung mit anorganischen oder organischen pharmazeutisch verträglichen Säuren weitere Salze gewinnen lassen.

Pharmazeutisch verträgliche Salze sind beispielsweise Salze mit anorganischen Säuren wie beispielsweise Salzsäure, Bromwasserstoffsäure, Schwefelsäure, Phosphorsäure oder Salpetersäure oder mit organischen Säuren wie Zitronensäure, Weinsäure, Maleinsäure, Fumarsäure, Bernsteinsäure, Äpfelsäure, Methansulfonsäure, Aminosulfonsäure, Essigsäure, Benzoesäure u.ä.

Die Verbindungen der Formel II des Formelblattes können, ausgehend von den Verbindungen der Formel IV gemäß Reaktionsschema I nach üblichen und dem Fachmann geläufigen chemischen Arbeitsmethoden hergestellt werden.

Die Verbindungen der Formel IV sind weitgehend literaturbekannt (DE-OS 25 34 689, DE-OS 28 39 266 und DE-OS 27 49 640). Die noch nicht bekannten können aus den in der Literatur beschriebenen Verbindungen der Formeln V und VI (DE-OS 25 37 070, DE-OS 28 35 760, DE-OS 28 38 851 und EP-A 103.142) gemäß dem Reatkionsschema II hergestellt werden.

Die Verbindungen der Formel III sind literaturbekannt (K.L. Howard, H.W. Stewart, E.A. Conroy und J.J. Denton, J. Org. Chem. 18, 1484 (1953)). 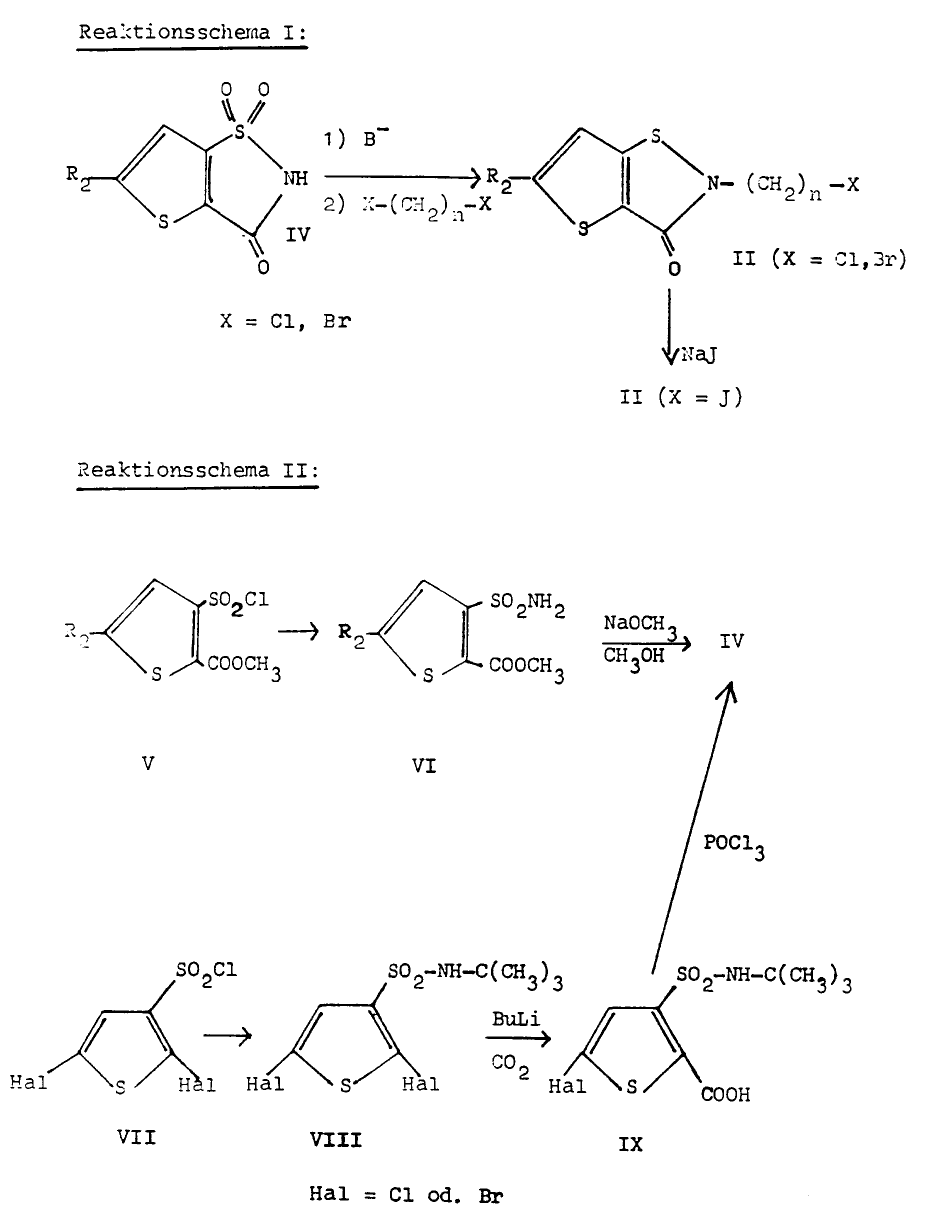
Die neuen Verbindungen der Formel I und ihre pharmazeutisch verwendbaren Salze zeigen in entsprechenden Tiermodellen ausgezeichnete anxiolytische Eigenschaften.

Aufgrund dieser pharmakologischen Eigenschaften können die neuen Verbindungen allein oder in Mischung mit anderen Wirksubstanzen in Form üblicher galenischer Zubereitungen zur Behandlung von verschiedenen Angstzuständen verwendet werden, ohne hypnotisch-sedative Nebenwirkungen zu verursachen.

Die Verbindungen der Formel I sind zur Verwendung am Menschen bestimmt und können auf übliche Weise, wie beispielsweise oral oder parenteral, verabreicht werden. Vorzugsweise werden sie oral verabreicht, wobei die Tagesdosis etwa 0,01 mg bis 10 mg/kg Körpergewicht beträgt, vorzugsweise 0,05 mg bis 0,5 mg/kg Körpergewicht. Bei intravenöser Gabe beträgt die Tagesdosis etwa 1 mcg bis 50 mcg/kg Körpergewicht, bevorzugt etwa 10 mcg/kg Körpergewicht. Der behandelnde Arzt kann jedoch, abhängig vom Allgemeinzustand und dem Alter des Patienten, der entsprechenden Substanz der Formel I, der Art der Krankheit und der Art der Formulierung, auch Dosen darüber oder darunter verschreiben.

Die Verbindungen der Formel I können allein oder in Verbindung mit anderen pharmazeutisch aktiven Substanzen verabreicht werden, wobei der Gehalt der Verbindungen der Formel I etwa zwischen 0,1 und 99 % liegt. Im allgemeinen liegen die phrmazeutisch aktiven Verbindungen in einer Mischung mit geeigneten inerten Hilfs- und/oder Trägerstoffen oder Verdünnnungsmitteln, wie z.B. pharmazeutisch unbedenklichen Lösungsmiteln, Gelatine, Gummi arabicum, Milchzucker, Stärke, Magnesiumstearat, Talk, pflanzlichen Ölen, Polyalkylenglykol, Vaseline und dergleichen vor.

Die pharmazeutischen Präparate können in fester Form, beispielsweise als Tabletten, Dragees, Suppositorien, Kapseln und dergleichen, in halbfester Form, beispielsweise als Salben oder in flüssiger Form, beispielsweise als Lösungen, Suspensionen oder Emulsionen, vorliegen. Gegebenenfalls sind sie sterilisiert und enthalten Hilfsstoffe, wie Konservierungs-, Stabilisierungs- oder Emulgiermittel, Salze zur Veränderung des osmotischen Druckes und dergleichen.

Insbesondere können pharmazeutische Präparate die erfindungsgemäßen Verbindungen in Kombination mit anderen therapeutisch wertvollen Stoffen enthalten. Mit diesen können die erfindungsgemäßen Verbindungen beispielsweise zusammen mit den oben angegebenen Hilfs- und/oder Trägerstoffen oder Verdünnungsmitteln zu Kombinationspräparaten formuliert werden.

Die in den folgenden Beispielen verwendeten Abkürzungen bedeuten:
- Ti: Thienoisothiazol
- Pyr: Pyrimidin
- Pip: Piperazin

### Beispiel 1:

### 5-Chlor-2-(4-(4-(2-pyrimidinyl)-1-piperazinyl)butyl)thieno-(2,3-d)isothiazol-3(2H)-on-1,1-dioxid

5,5 g (13,6 mmol) 2-(4-Jodbutyl)-5-chlor-thieno(2,3-d)isothiazol-3(2H)-on-1,1-dioxid werden mit 25 ml absolutem Dimethylformamid versetzt und die Lösung auf 40 °C erwärmt. Dann werden 2,23 g (13,6 mmol) 1-(2-Pyrimidinyl)piperazin in abs. Dimethylformamid bei 60 °C gelöst und innerhalb einer Minute zugegeben. Nach 45 Minuten bei 60 °C wird das Lösungsmittel abgedampft und der ölige orange Rückstand in 25 ml Methylenchlorid aufgenommen. Die Methylenchloridphase wird noch zweimal mit je 20 ml Wasser ausgeschüttelt und anschließend achtmal mit insgesamt 130 ml 2n Salzsäure extrahiert. Die saure, wäßrige Phase wird mit festem Natriumbikarbonat neutralisiert (pH-Wert = 7,5) und dann viermal mit je 25 ml Methylenchlorid ausgeschüttelt. Die vereinigten organischen Phasen werden über Natriumsulfat getrocknet, filtriert und eingedampft. Das erhaltene Rohprodukt (4,0 g; 57 % d.Th.) wird in 45 ml Isopropanol in der Siedehitze gelöst und wenig unlösliches Nebenprodukt (70 mg) heiß abfiltriert. Die Mutterlauge wird im Tiefkühlschrank unter mehrmaligem Anreiben auskristallisieren gelassen, dann das gelbe Produkt abgenutscht und dreimal mit eiskaltem Isopropanol digeriert. Das erhaltene Rohprodukt (3,3 g; 7,67 mmol) wird in 35 ml Aceton in der Siedehitze gelöst, filtriert und nach dem Abkühlen unter Rühren mit 0,93 g (7,67 mmol) 29,2 %iger methanolischer Salzsäure vesetzt. Das Hydrochlorid wird im Tiefkühlschrank unter mehrmaligem Anreiben auskristallisieren gelassen, abgesaugt und mit wenig eiskaltem Aceton dreimal digeriert. Die bei 40 °C/20 mbar getrockneten 2,93 g Hydrochlorid werden in 45 ml Wasser suspendiert, mit gesättigter Natriumbikarbonatlösung auf einen pH-Wert von 7,5 gebracht und viermal mit je 30 ml Methylenchlorid extrahiert. Die vereinigten organischen Phasen werden über Natriumsulfat getrocknet, mit Aktivkohle versetzt, abfiltriert und eingedampft (2,75 g). Die Endreinigung erfolgt säulenchromatographisch (KG 60; 50:1; Eluens: Diethylether). Das Produkt wird erneut aus 25 ml Isopropanol umkristallisiert.
Ausbeute: 2,25 g schwach gelbe Kristalle (38 % d.Th.)
Fp: 134 - 135,5 °C (Isopropanol)
Mikroelementaranalyse:

| C₁₇H₂₀N₅ClO₃S₂ MG = 441,96 | | | |
|---|---|---|---|
| | C | H | N |
| berechnet | 46,20 | 4,56 | 15,85 |
| gefunden | 46,04 | 4,62 | 15,70 |

¹H-NMR: (CDCl₃)
delta (ppm): 8,29 (d; J=4,9 Hz; 2H, Pyr-H₄ und H₆), 7,28 (s; 1H; Ti-H₆), 6,47 (t; J=4,9 HZ; 1H; Pyr-H₅), 3,90 - 3,70 (m; 6H; Pip-H₃ und H₅, Ti-CH₂-), 2,56 - 2,36 (m; 6H; Pip-H₂ und H₆, -CH₂Pip), 2,00 - 1,50 (m; 4H; Ti-C-CH₂- und -CH₂-C-Pip-).

Das Ausgangsprodukt kann wie folgt hergestellt werden:

### 2-((4-Brombutyl)-5-chlor-thieno(2,3-d)isothiazol-3(2H)on-1,1-dioxid

15 g (67,1 mmol) 5-Chlor-thieno(2,3-d)isothiazol-3(2H)-on-1,1-dioxid werden in 100 ml absolutem Dimethylformamid gelöst. Dann werden 2,82 g (70,5 mmol) 60 %ige Natriumhydridsuspension viermal mit absolutem Benzol gewaschen und unter Eiskühlung und starkem magnetischen Rühren so langsam zur DMF-Lösung zugegeben, daß die Temperatur nicht über 15 °C steigt. Das Reaktionsgemisch wird nach 15 minütigem Rühren bei Raumtemperatur auf 60 °C erhitzt und innerhalb von 30 Minuten mit 43,5 g (202 mmol) 1,4-Dibrombutan versetzt. Nach drei Stunden bei 60 °C wird die Lösung bei 70 °C/1,5 mbar eingedampft. Das verbleibende gelbe Öl wird in 40 ml gesättigter Natriumbikarbonatlösung suspendiert und dreimal mit je 50 ml Methylenchlorid extrahiert. Dann werden die vereinigten organischen Phasen zweimal mit je 50 ml gesättigter Natriumbikarbonatlösung und zweimal mit insgesamt 110 ml Wasser rückgeschüttelt. Die organische Phase wird über Natriumsulfat getrocknet, mit Aktivkkohle versetzt, filtriert und eingedampft. Das Rohprodukt wird in 100 ml Diethylether in der Siedehitze gelöst ung 500 mg farbloses Nebenprodukt abgesaugt. Nach dem Eindampfen des Lösungsmittels bleiben 19,74 g festes Rohprodukt zurück, das ohne weitere Reinigung in den nächsten Reaktionsschritt eingesetzt werden kann. 0,7 g werden einer säulenchromatographischen Reinigung unterzogen (KG 60; 40:1; Eluens: Methylenchlorid; Ausbeute: 0,62 g).
- Ausbeute:: 17,48 g farblose Kristalle (73 % d.Th.)
- Fp:: 75 - 76 °C (Diethylether)

### 2-(4-Jodbutyl)-5-chlor-thieno(2,3-d)isothiazol-3(2H)on-1,1-dioxid

2,3 g (15,3 mmol) Natriumjodid werden in 70 ml absolutem Aceton gelöst und 5,5 g (15,3 mmol) 2-(4-Brombutyl)-5-chlor-thieno-(2,3-d)isothiazol-3(2H)-on-1,1-dioxid auf einmal zugegeben. Unter starkem magnetischen Rühren wird 90 Minuten unter Rückfluß erhitzt, wobei ein farbloser, voluminöser Niederschlag entsteht. Das Reaktionsgemisch wird eingedampft, in 40 ml Methylenchlorid aufgenommen und zweimal mit je 60 ml gesättigter Natriumbisulfitlösung extrahiert. Die Methylenchloridphase wird mit 25 ml Wasser rückgeschüttelt. Die organische Phase wird über Natriumsulfat getrocknet, filtriert und eingedampft. Das Rohprodukt kann direkt in die nächste Stufe eingesetzt werden. 1 g Rohprodukt wird säulenchormatographisch gereinigt (KG 60; 30:1; Eluens: Methylenchlord:Petrolether = 2:1; Ausbeute: 0,94 g).
- Ausbeute:: 5,57 g schwach gelbe Kristalle (90 % d.Th.)
- Fp:: 86 - 87 °C (Methylenchlorid)

### Beispiel 2: (Vergleichsbeispiel)

Analog zu Beispiel 1 wurde folgende Vergleichssubstanz hergestellt:

### 2-(4-(4-(2-Pyrimidinyl)-1-piperazinyl)butyl)-thieno(2,3-d)-isothiazol-3(2H)-on-1,1-dioxid

- Fp:: 117 - 118 °C (Isopropanol)

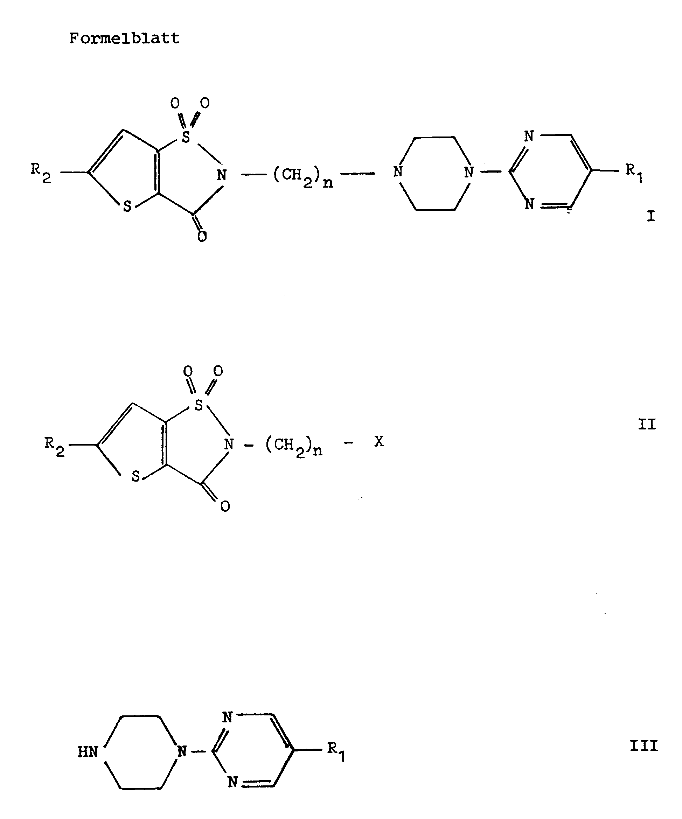

## Patentansprüche (Patentansprüche für folgende(n) Vertragsstaat(en): AT, BE, CH, DE, FR, GB, IT, LI, LU, NL, SE)

1. Basisch substituierte 5-Halogen-Thienoisothiazol-3(2H)-on-1,1-dioxide der Formel I 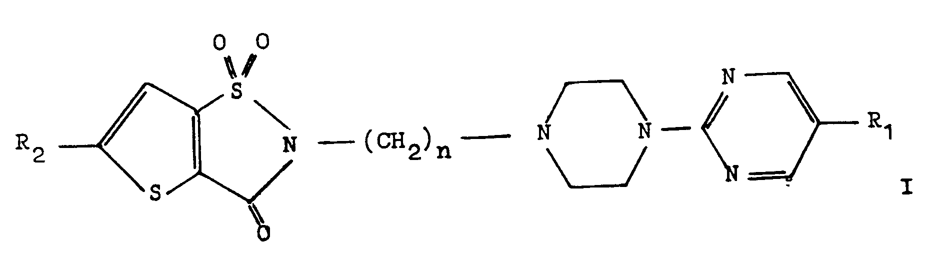 in der
R₁ Wasserstoff, (C₁ - C₄)-Alkyl oder Halogen,
R₂ Halogen und
n eine ganze Zahl 2 bis 6 darstellt und ihre pharmazeutisch verträglichen Säureadditionssalze.

2. 5-Chlor-2-(4-(4-(2-pyrimidinyl)-1-piperazinyl)butyl)thieno-(2,3-d)isothiazol-3(2H)-on-1,1-dioxid.

3. Verfahren zur Herstellung von Verbindungen der in Anspruch 1 definierten Formel I des Formelblattes, dadurch gekennzeichnet, daß man eine Verbindung der Formel II 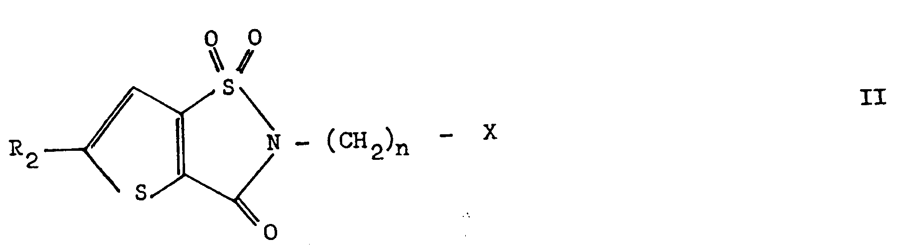 in der R₂ und n wie oben definiert sind und X Halogen bedeutet, mit einer Verbindung der Formel III 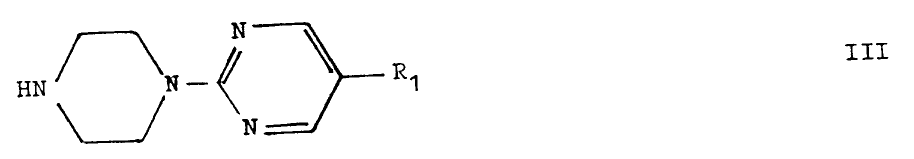 in der R₁ Wasserstoff, (C₁ -
C₄)-Alkyl oder Halogen bedeutet, umsetzt und eine so erhaltene Base der Formel I erwünschtenfalls in ein pharmazeutisch verträgliches Säureadditionssalz überführt.

4. Pharmazeutische Präparate enthaltend Verbindungen der Formel I des Formelblattes oder ein pharmazeutisch verträgliches Salz davon in Kombination mit üblichen galenischen Hilfs- und/oder Trägerstoffen oder Verdünngungsmitteln.

5. Pharmazeutische Präparate, enthaltend Verbindungen der allgemeinen Formel I des Formelblattes oder pharmazeutisch verträgliche Salze davon in Kombination mit anderen therapeutisch wertvollen Wirkstoffen sowie üblichen galenischen Hilfs- und/oder Trägerstoffen oder Verdünnungsmitteln.

## Patentansprüche (Patentansprüche für folgende(n) Vertragsstaat(en): ES, GR)

1. Verfahren zur Herstellung basisch substituierter 5-Halogen-Thienoisothiazol-3(2H)-on-1,1-dioxide der Formel I 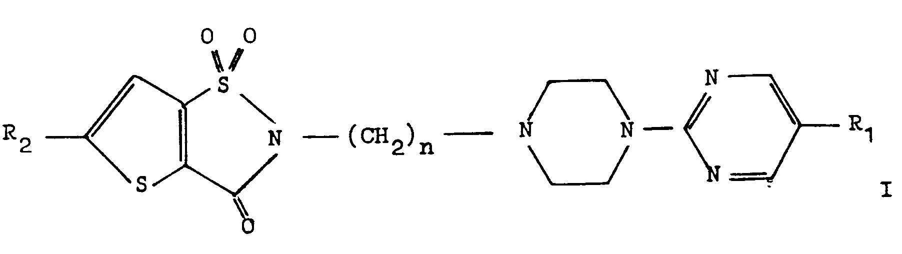 in der
R₁ Wasserstoff, (C₁ - C₄)-Alkyl oder Halogen,
R₂ Halogen und
n eine ganze Zahl 2 bis 6 darstellt und von deren pharmazeutisch verträglichen Säureadditionssalzen, dadurch gekennzeichnet, daß man eine Verbindung der Formel II 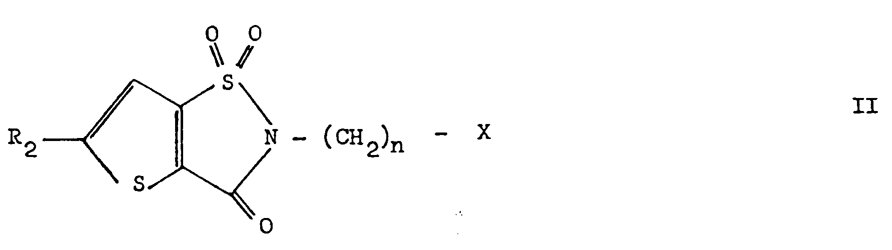 in der R₂ und n wie oben definiert sind und X Halogen bedeutet, mit einer Verbindung der Formel III 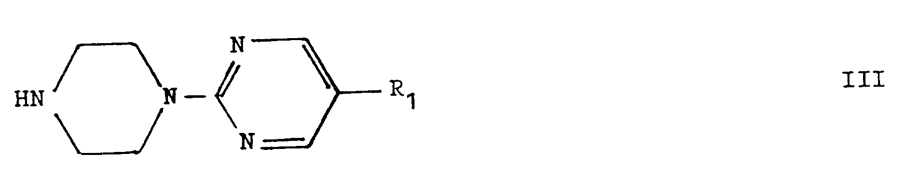 in der R₁ Wasserstoff, (C₁ -
C₄)-Alkyl oder Halogen bedeutet, umsetzt und eine so erhaltene Base der Formel I erwünschtenfalls in ein pharmazeutisch verträgliches Säureadditionssalz überführt.

2. Verfahren zur Herstellung von Verbindungen der in Anspruch 1 definierten Formel I des Formelblattes und ihrer pharmazeutisch verträglichen Säureadditionssalze, dadurch gekennzeichnet, daß man die Umsetzung in einem organischen Verdünnungsmittel bei einer Temperatur von 20 bis 150 °C während einer Dauer von 30 Minuten bis 15 Stunden durchführt.

3. Verfahren zur Herstellung von Verbindungen der in Anspruch 1 definierten Formel I des Formelblattes und ihrer pharmazeutisch verträglichen Säureadditionssalze, dadurch gekennzeichnet, daß man die Umsetzung in Dimethylformamid bei 50 bis 70 °C während einer Dauer von 30 - 60 Minuten durchführt.

## Claims (Claims for the following Contracting State(s): AT, BE, CH, DE, FR, GB, IT, LI, LU, NL, SE)

1. Basic-substituted 5-halo-thienoisothiazol-3(2H)-one 1,1-dioxide of the formula I 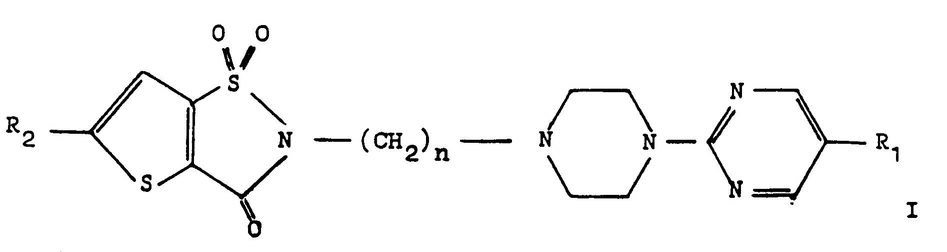 in which
R₁ denotes hydrogen, (C₁-C₄)-alkyl or halogen,
R₂ denotes halogen, and
n denotes an integer from 2 to 6, and the pharmaceutically acceptable acid-addition salts thereof.

2. 5-Chloro-2-(4-(4-(2-pyrimidinyl)-1-piperazinyl)butyl)thieno-(2,3-d)isothiazol-3(2H)-one 1,1-dioxide.

3. Process for the preparation of compounds of the formula I of the sheet of formulae defined in claim 1, characterized in that a compound of the formula II 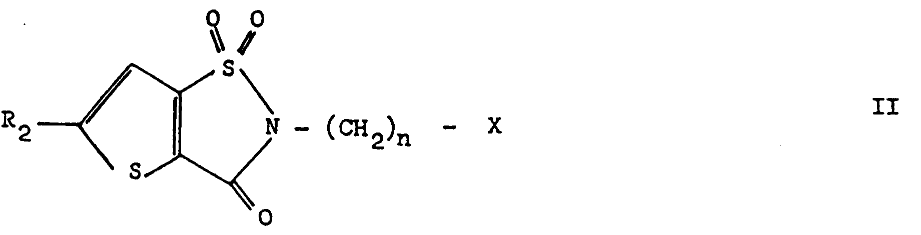 in which R₂ and n are as defined above and X denotes halogen, is reacted with a compound of the formula III 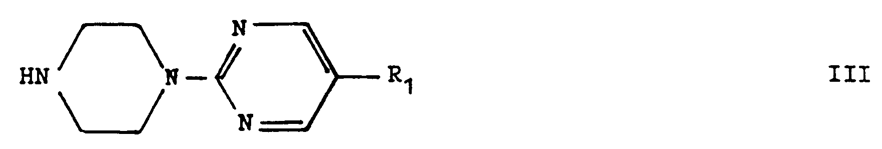 in which R₁ is hydrogen, (C₁-C₄)-alkyl or halogen and, if desired, a base of the formula I obtained is converted into a pharmaceutically acceptable acid-addition salt.

4. Pharmaceutical preparations containing compounds of the formula I of the sheet of formulae or a pharmaceutically acceptable salt thereof, in combination with customary gallinic adjuvants or carriers or diluents.

5. Pharmaceutical preparations containing compounds of the general formula I of the sheet of formulae or pharmaceutically acceptable salts thereof, in combination with other therapeutically useful active compounds and customary gallinic adjuvants and/or carriers or diluents.

## Claims (Claims for the following Contracting State(s): ES, GR)

1. Process for the preparation of basic-substituted 5-halo-thienoisothiazol-3(2H)-one 1,1-dioxide of the formula I 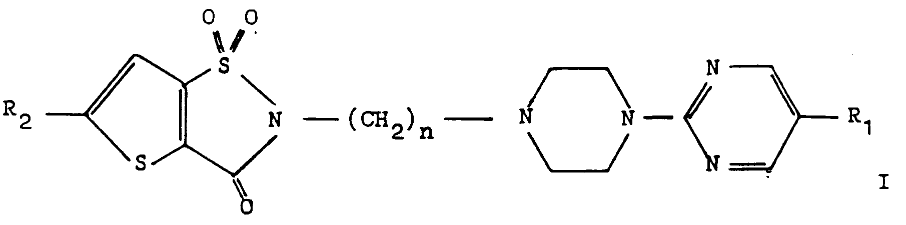 in which
R₁ denotes hydrogen, (C₁-C₄)-alkyl or halogen,
R₂ denotes halogen, and
n denotes an integer from 2 to 6, and pharmaceutically acceptable acid-addition salts thereof characterized in that a compound of the formula II 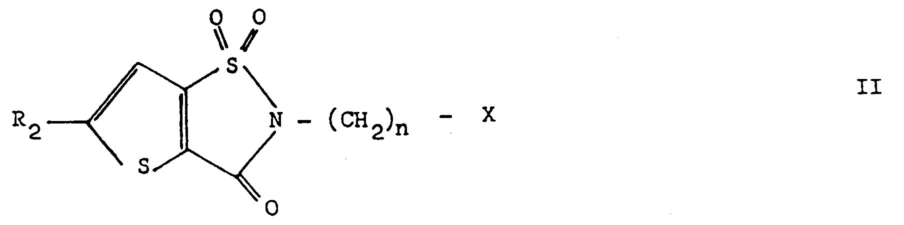 in which R₂ and n are as defined above and X denotes halogen, is reacted with a compound of the formula III 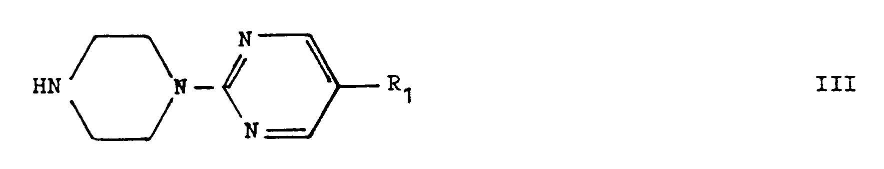 in which R₁ is hydrogen, (C₁-C₄)-alkyl or halogen and, if desired, a base of the formula I obtained is converted into a pharmaceutically acceptable acid-addition salt.

2. Process for the preparation of compounds of the formula I of the sheet of formulae defined in claim 1 and their pharmaceutically acceptable acid-addition salts, characterized in that the reaction is carried out in an organic diluent at a temperature of from 20 to 150°C for a period of from 30 minutes to 15 hours.

3. Process for the preparation of compounds of the formula I of the sheet of formulae defined in claim 1 and their pharmaceutically acceptable acid-addition salts, characterized in that the reaction is carried out in dimethylformamide at from 50 to 70°C for a period of 30-60 minutes.

## Revendications (Revendications pour l'(les) Etat(s) contractant(s) suivant(s): AT, BE, CH, DE, FR, GB, IT, LI, LU, NL, SE)

1. Des 5-halo-thiénoisothiazol-3(2H)-on-1,1-dioxydes à substituant basique de formule I 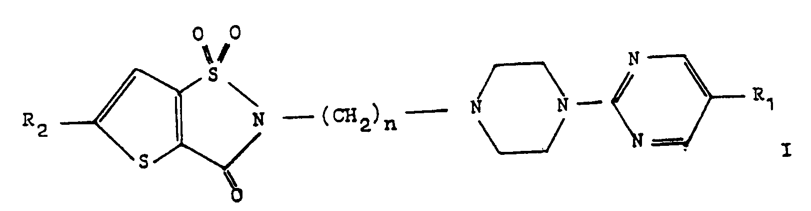 dans laquelle
R₁ représente un hydrogène, un alkyl en C₁-C₄ ou un halogène,
R₂ représente un halogène et
n un nombre entier de 2 à 6, leurs sels d'addition d'acide compatibles sur le plan pharmaceutique.

2. Le 5-chloro-2-(4-(4-(2-pyrimidinyl)-1-pipérazinyl)butyl)thiéno-(2,3-d)-isothiazol-3(2H)-on-1,1-dioxyde.

3. Un procédé pour la préparation de composés de la formule I de la page de formules définies à la revendication I, caractérisé en ce qu'un composé de formule II 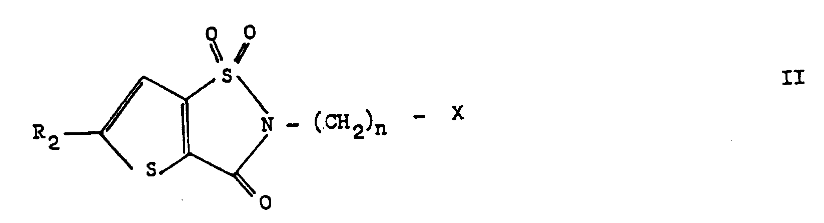 dans laquelle R₂ et n sont définis comme ci-dessus et X représente un halogène, entre en réaction avec un composé de formule III 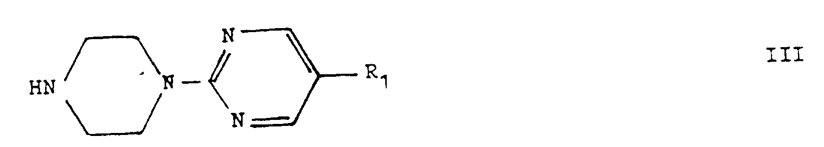 dans laquelle R₁ représente un hydrogène, un alkyle en C₁-C₄ ou un halogène et que l'on transforme, si on le souhaite, la base de formule I ainsi obtenue en un sel d'addition d'acide compatible au plan pharmaceutique.

4. Des compositions pharmaceutiques contenant les composés de la formule I de la page de formules ou leurs sels compatibles au plan pharmaceutique se trouvant en mélange avec des excipients et/ou des supports ou des diluants galéniques courants.

5. Des compositions pharmaceutiques contenant les composés de la formule générale I de la page de formules ou leurs sels compatibles au plan pharmaceutique, en mélange avec d'autres substances actives au plan thérapeutique, de même qu'avec des excipients et/ou supports ou des diluants galéniques courants.

## Revendications (Revendications pour l'(les) Etat(s) contractant(s) suivant(s): ES, GR)

1. Procédé pour la préparation de 5-halo-thiénoisothiazol-3(2H)-on-1,1-dioxydes à substituant basique de formule I 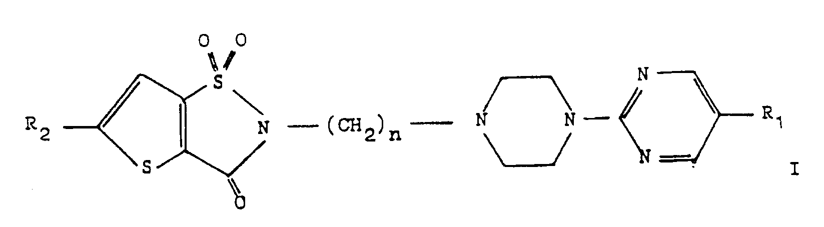 dans laquelle
R₁ représente un hydrogène, un alkyle en C₁-C₄ ou un halogène,
R₂ représente un halogène et
n un nombre entier de 2 à 6 et leurs sels d'addition d'acide compatibles sur le plan pharmaceutique, caractérisé en ce que un composé de formule II 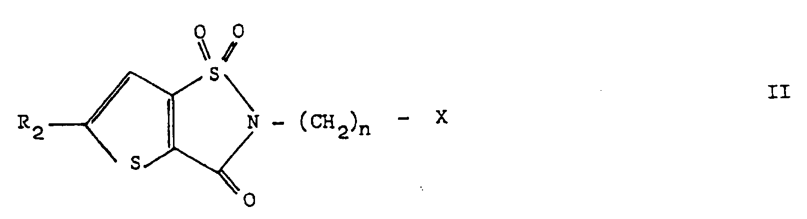 dans laquelle R₂ et n sont définis comme ci-dessus et X représente un halogène, entre en réaction avec un composé de formule III 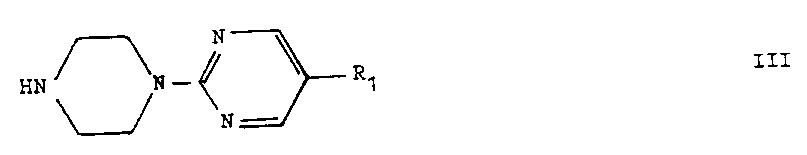 dans laquelle R₁ représente un hydrogène, un alkyl en C₁-C₄ ou un halogène et que l'on transforme, si on le souhaite, la base de formule I ainsi obtenue en un sel d'addition d'acide compatible au plan pharmaceutique.

2. Un procédé pour la préparation de composés de la formule I de la page de formules définis à la revendication 1 et leurs sels compatibles au plan pharmaceutique, caractérisé en ce que la réaction est réalisée dans un diluant organique à une température de 20 à 150 °C pendant une durée de 30 minutes à 15 heures.

3. Un procédé pour la préparation de composés de la formule I de la page de formules définis à la revendication 1 et leurs sels compatibles au plan pharmaceutique, caractérisé en ce que la réaction est réalisée dans du diméthylformamide à une température de 50 à 70 °C pendant une durée de 30 - 60 minutes.
